# EUROPEAN PATENT APPLICATION

(11) **EP 2 902 028 A1**
(43) Date of publication of application: **05.08.2015**
(21) Application number: 13841408.1
(22) Date of filing: 27.09.2013
(51) Int. Cl.: A61K 31/4748, A61K 31/506, A61P 35/00, A61P 35/02

(54) **DRUG COMPOSITION FOR TREATING TUMORS AND APPLICATION THEREOF**

(30) Priority: 28.09.2012 WO PCT/CN2012/082386
(71) Applicant: Hangzhou Bensheng Pharmaceutical Co., Ltd., Hangzhou, Zhejiang 310051 (CN)
(72) Inventor: XU, Rongzhen, Hangzhou Zhejiang 310012 (CN); RONG, Frank, Hangzhou Zhejiang 310012 (CN); XIE, Fuwen, Longyan Fujian 364200 (CN)
(74) Representative: Cabinet Plasseraud
(86) International application number: PCT/CN2013/084537
(87) International publication number: WO 2014/048377

(57) **Abstract**

The present invention belongs to the field of medicine and pharmaceutical chemistry, specifically relates to novel antitumor pharmaceutical combinations, and particularly relates to pharmaceutical combinations of bisbenzylisoquinoline alkaloids (e.g. berbamine and tetrandrine) and imatinib mesylates and their use in treating tumors.

## Description

### Technical Field

The present invention belongs to the field of medicine and pharmaceutical chemistry, relates to novel antitumor pharmaceutical combinations, and particularly relates to pharmaceutical combinations of bisbenzylisoquinoline alkaloids and imatinib mesylates and their use in treating tumors.

### Background of the Invention

Bisbenzylisoquinoline alkaloids are a class of very important natural products. They are derived from a wide range of medicinal materials, and can be separated, extracted, and characterized with mature processes. A plurality of these compounds with determined structures have been used as medicines for clinical applications, such as berbamine, tetrandrine, and fangchinoline.

Berbamine (BBM), also known as 6,6',7-trimethoxy-2,2'-dimethylberbaman-12-ol, is a bisbenzyl isoquinoline alkaloid extracted from Chinese herbal plants of berberis.

Berbamine has the effect of stimulating myeloid cell proliferation, improving the level of hematopoietic stem cell colony stimulating factor (GCSF), promoting proliferation of bone marrow hematopoietic stem cells and myeloid progenitor cells as well as their differentiation to granulocytes, and promoting proliferation of leukocytes (LIN Chuanrong, et al., The clinical observations on the treatment of chemotherapy-induced leukopenia with Shengbaijiao (berbamine), Prepared Chinese Medicine, 1994, 16 (7): 29).

Berbamine inhibits the proliferation of prostate cancer PC-3 cells by means of inducing cell apoptosis and affecting the cell cycles, and exhibits time- and concentration-dependencies (SUN Peng et al, Induction of apoptosis by berbamine in androgen-independent prostate cancer cell PC-3 in vitro, Chin J Exp Surg, August 2007, Vol 24, No. 8, pp 957).

Berbamine has significant anti-proliferation effect and specific apoptosis-inducing effect on K562 cells in vitro, and exhibits time-dependency. In a nude mouse bearing the tumor, berbamine also shows significant growth inhibiting effect to the K562 cells, and can particularly reduce the expression level of the tumor histocyte bcr/abl mRNA (WU Dong et al, Effects of Berbamine on K562 Cells and Its Mechanisms in vitro and in vivo, Journal of Experimental Hematology 2005, 13 (3): 373-378).

There are also research reports about the mechanisms of berbamine inducing the apoptosis of human leukemia Jurkat cells. Results show that berbamine can selectively inhibit the apoptosis of human leukemia Jurkat cells, arrest the cell cycle at S phase, and increase the protein expression of the cell caspase-3. With the active drug concentration increases from 0.5 µg/mL to 10 µg/mL, the cell viability decreases from 93.69% to 14.85%. Within this range of drug concentration, berbamine has no evident cytotoxic effect on the peripheral blood leucocyte of a normal human being (HUANG Zhiyu et al, Experimental Study of Berbamine Inducing Apoptosis in Human Leukemia Jurkat Cells, China Cancer, 2007, 16(9), 722).

Fangchinoline (FAN), also known as hanfangichin, is a bisbenzyl isoquinoline alkaloid extracted from Chinese herbal powder of fangji root. Research in recent years has discovered that it also has significant antitumor effects, such as significant inhibitory effect on W-256, Ehrlich ascites carcinoma, S-45, as well as inhibitory effects on KB cells and Hela cells, and strong inhibitory effects on human transplanted liver cancer cell lines 7402 and 7405.

Studies have reported the inhibitory effect of fangchinoline on human colon cancer cell line LoVo. Tumor-bearing nude mice were administered with fangchinoline in the experiments continuously for 60 days, and the results showed that, as compared with the control group, the treated group exhibited significantly reduced and slow-growing tumor and lower microvessel density. In addition, during the administration, the mice did not show any adverse reaction, thus indicating the inhibitory effect of fangchinoline on tumor in vivo with minor side effects (WANG Zhenjun et al, Use of fangchinoline in angiogenesis inhibiting medicaments, [P] CN 1511528A. 2004-07-14).

Changdong Wang, et al. studied the inhibitory effect of fangchinoline on human prostate cancer cell PC3. Results showed that the inhibitory effect of fangchinoline on PC3 cells was dosage- and time-dependent. Studies on cell cycle progress showed that the inhibitory effect of fangchinoline on PC3 cells was relevant to the increase of G1/S phase cells. In addition, fangchinoline had in vivo antitumor activity, reduced the tumor volume and promoted the apoptosis thereof, and inhibitory effect on the transplanted tumor PC3 in nude mice (Changdong Wang, et al. Fangchinoline induced G1/S arrest by modulating expression of p27, PCNA, and cyclin D in human prostate carcinoma cancer PC3 cells and tumor xenograft. Biosci. Biotechnol. Biochem. 2010, 74(3):488-493).

TANG Yalin et al studied the combination of G-quadruplex DNA and fangchinoline to increase the stability of G-quadruplex, thus weakening the ability of cell proliferation and promoting its apoptosis. In vitro anticancer cell proliferation experiments showed that the combination of G-quadruplex DNA and fangchinoline has better inhibitory effect on human leukemia cell line HL-60, human gastric carcinoma cell line BGC-823, human hepatocellular carcinoma cell line Bel-7402 and human nasopharyngeal carcinoma cell line KB (TANG Yalin et al, New use of bisbenzylisoquinoline alkaloid, [P] CN 101371839A. 2009-02-25).

Tetrandrine, or TTD, with the chemical formula of 6,6',7,12-tetramethoxy -2,2'dimethylberbamine, is a bisbenzylisoquinoline alkaloid extracted from the root block of the Chinese herbal fangji.

Tetrandrine exhibits inhibition on the proliferation of cervical cancer HeLa cells. The studies use the MTT method to detect inhibition on the proliferation of the cervical cancer HeLa cell lines by tetrandrine in various concentrations and at different time. The cell apoptosis was detected by flow cytometry and laser scanning confocal microscope. As is shown by the experiments, tetrandrine exhibits inhibition on proliferation of the cervical cancer HeLa cells, which exhibits time- and concentration- dependencies. (ZHU Kexiu et al., Qualitative and quantitative studies on tetrandrine-induced apoptosis of cervical cancer cells, Journal of Xi'an Jiaotong University (Medical Sciences Edition), 2010, 31 (1), 102).

Tetrandrine can inhibit the proliferation of hepatoma cells. After action of tetrandrine on the hepatoma cells, reactive oxygen species (ROS) are generated within 2 hours, and the production of ROS increases significantly with the increase of dose. It is suggested that tetrandrine may generate reactive oxygen species by interfering with the mitochondrial function, which causes the cell lipid peroxidation, damages the DNA molecules or regulates related genes of apoptosis, thereby inhibiting the proliferation of hepatoma cells. (Jing Xubin et al., Experimental studies on the tetrandrine-induced oxidative damage of hepatoma cells, Journal of Clinical Hepatology, 2002, 18 (6), 366).

In addition, there are reports on the significant inhibitory effect of tetrandrine in vitro on the growth of human neuroblastoma TGW. Research shows that, with the increase of dose, the inhibition gradually increases; with the increase ofaction time, inhibitory effect also obviously increases, thus showing excellent dosage- and time-correlation (LI Weisong et al., Clinical Journal of Pediatrics, Experimental studies on tetrandrine induced neuroblastoma cell line TDW apoptosis, 2006, 24 (6), 512).

Imatinib, i.e. 4-[(4-methyl-1-piperazinyl)methyl]-N-[4-methyl-3-4-(3-pyridyl)-2-pyrimidinyl]amino] phenyl]benzamide (US 5521184), has a mesylate being the active ingredient of the drug Gleevec ® (Glivec ®). Imatinib mesylate is a protein tyrosine kinase inhibitor, can inhibit Bcr-Abl tyrosine kinases in cell level both in vitro and in vivo, can selectively inhibit Bcr-Abl positive cell lines, Ph chromosome positive chronic myelogenous leukemia and acute lymphoblastic leukaemia patient fresh cell proliferation, and induce apoptosis thereof. In addition, imatinib mesylate can also inhibit platelet-derived growth factor (PDGF) receptor, stem cell factor (SCF), c-Kit receptor tyrosine kinase, thereby inhibiting the cell behavior mediated by PDGF and stem cell factor.

Imatinib mesylate is developed by Novartis and is used as medicament for patients with granulocytic leukemia who failed previous interferon alpha-based therapies in blastocyte crisis stadium, chronic stadium, and accelerated stadium. In February 2002, the FDA approved the second indication--for gastrointestinal stromal cell tumors. It has become the first-line therapy for chronic myeloid leukemia.

Imatinib's applications as antitumor drugs are recorded in EP-A-0564409 and the counterparts of this application in many other countries (such as US 5521184). Imatinib mesylate is described in US patent US 6894051. According to this literature, this compound inhibits the PFGF-receptor kinase, and also inhibits the protein kinase C in warm-blood animals, and thus it can be used for the treatment of various cancers.

On the other hand, drug combination has become the prevalent method of clinical treatment of AIDS. This method has many advantages, for example: (a) inhibiting HIV replication at multiple target sites and delaying the clinical progression of HIV infection; (b) reducing the probability of HIV drug resistance occurrence to the minimum; (c) significantly increasing the AIDS patients' quality of life and survival rate. The cocktail therapy proposed by Dr. David Ho is performed by the use of three or more antiviral drugs in combination for the treatment of AIDS. The application of this therapy can reduce the drug resistance from using one single drug, inhibit viral replication to the maximum, and cause partial or full recovery of the immune function of the damaged bodies, thus delaying the progression of disease, prolonging patients' lives, and improving the quality of life. More and more scientists believe that mixed drug therapy is the most effective treatment to AIDS by both inhibiting HIV reproduction and preventing the emergence of drug-resistant virus in vivo.

In recent years, for other diseases, researchers have called the analogous pharmaceutical combination therapy a "cocktail therapy".

Because of the diversity of the patients and complexity of tumors, bisbenzylisoquinoline alkaloids and imatinib mesylates have shown their limitations while used alone (injection or oral), such as poor selectivity, low inhibition rate or low cure rate, high side effects or toxicity resulted from high doses, and the like. In order to solve the above problems, the present invention provides the combination of bisbenzylisoquinoline alkaloids and imatinib mesylates to improve the efficacy, selectivity and the cure rate in the treatment of tumors. At present, it has not yet been reported that the combination of bisbenzylisoquinoline alkaloids and imatinib mesylates is used for the treatment of tumor to improve the drug efficacy or cure rate. The objective of the present invention is to provide a "cocktail" or a composite medicine for treating tumors.

### Summary of the invention

One object of the present invention is to provide an antitumor pharmaceutical combination charaterzied by a bisbenzylisoquinoline alkaloid of Formula (I) and an imatinib mesylate (II) and a pharmaceutically acceptable salt thereof, 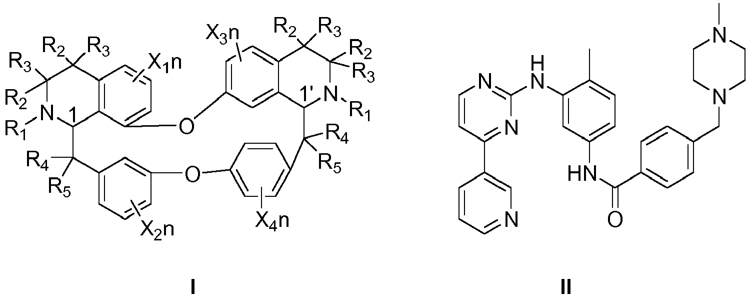 in Formula (I),
R₁ is H or linear or branched alkyl comprising 1 to 10 carbon atoms;
R₂ and R₃ each independently is H, substituted acyl, linear or branched alkyl, wherein the alkyl can be interrupted by O, N, S heteroatoms; or R₂ and R₃ together represents O or S;
R₄ and R₅ each independently is H, substituted acyl, linear or branched alkyl, wherein the alkyl can be interrupted by O, N, or S heteroatoms; or R₄ and R₅ together represents O or S;
X₁, X₂, X₃, and X₄ can be identical or different, each of which independently is halogen atoms, hydroxyl, or linear or branched alkyl comprising 1 to 10 carbon atoms, or alkoxy, or acyloxy, and n is an integer of from 0 to 4;
C(1) and C(1') are of a stereoisomer configuration selected from RR, SS, 1S1'R, 1R1'S.

The bisbenzylisoquinoline alkaloid of Formula (I) can be selected from the following:

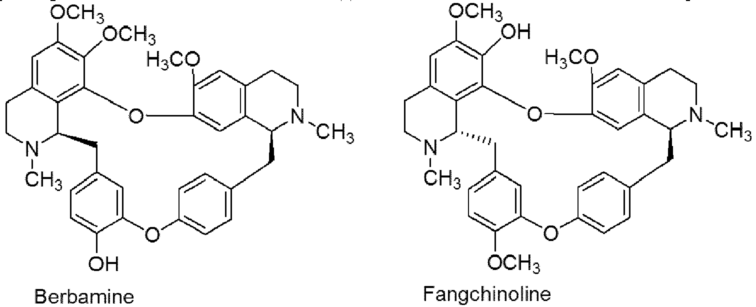

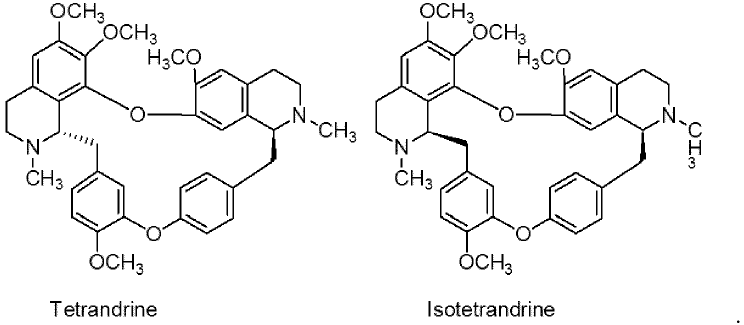

A second object of the present invention is to provide a suitable combination of the bisbenzylisoquinoline alkaloid of Formula (I) and the imatinib mesylate (II) as an antitumor medicament. The synergistic effect between the two, particularly the pharmaceutical combination of berbamine and imatinib mesylate, as well as the pharmaceutical combination of tetrandrine and imatinib mesylate, achieves better therapeutic effects.

A third object of the present invention is to provide the use, particularly the antitumor clinical use, of the pharmaceutical combination of the present invention. Correspondingly, the present invention provides a method for treating a subject suffering from tumor, comprising administrating to the subject in need thereof an effective amount of the pharmaceutical combination of the present invention, wherein the tumor is particularly selected from leukemia, multiple myeloma, lymphoma, liver cancer, gastric cancer, breast cancer, cholangiocellular carcinoma, pancreatic cancer, lung cancer, colorectal carcinoma, osteosarcoma, melanoma, human cervical cancer, glioma, nasopharyngeal carcinoma, laryngeal carcinoma, esophageal cancer, middle ear tumor, and prostate cancer.

### Brief description of the drawings

Figure 1A shows the inhibitory effect of IM combined with BBM on leukemia cell K562/ADR (see Table 1 for compound dosages);
Figure 1B shows the inhibitory effect of IM combined with TTD on leukemia cell KCL-22M (see Table 1 for compound dosages);
Figure 2 shows the dynamic variation of the effect of the pharmaceutical combination of BBM and IM on the body weight of BALB/c-nu nude mice;
Figure 3 shows the dynamic variation of the effect of the pharmaceutical combination of BBM and IM on the leukemia transplanted tumor of BALB/c-nu nude mice;
Figure 4 shows the effect of the pharmaceutical combination of BBM and IM on the leukemia transplanted tumor size of BALB/c-nu nude mice;
Figure 5 shows the effect of the pharmaceutical combination of BBM and IM on the leukemia transplanted tumor weight of BALB/c-nu nude mice;
Figure 6 shows the inhibitory effect of the pharmaceutical combination of BBM and IM on the leukemia transplanted tumor of BALB/c-nu nude mice;
Figure 7 shows the dynamic variation of the effect of the pharmaceutical combination of TTD and IM on the body weight of BALB/c-nu nude mice;
Figure 8 shows the dynamic variation of the effect of the pharmaceutical combination of TTD and IM on the leukemia transplanted tumor of BALB/c-nu nude mice;
Figure 9 shows the effect of the pharmaceutical combination of TTD and IM on the leukemia transplanted tumor size of BALB/c-nu nude mice;
Figure 10 shows the effect of the pharmaceutical combination of TTD and IM on the leukemia transplanted tumor weight of BALB/c-nu nude mice;
Figure 11 shows the inhibitory effect of the pharmaceutical combination of TTD and IM on the leukemia transplanted tumor of BALB/c-nu nude mice.

### Detailed description of the invention

The present invention provides an antitumor pharmaceutical combination, which is charaterzied by comprising a bisbenzylisoquinoline alkaloid of Formula (I) and an imatinib mesylate (II) 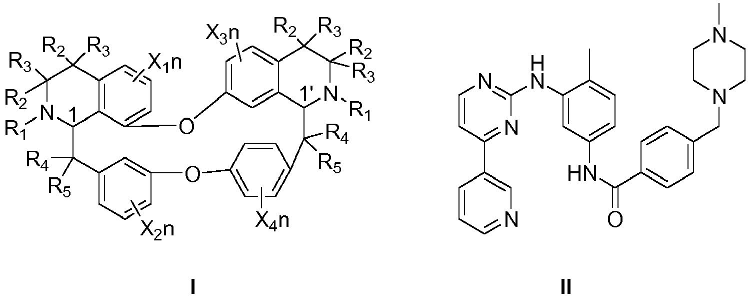 in Formula (I),
R₁ is H or linear or branched alkyl comprising 1 to 10 carbon atoms;
R₂ and R₃ each independently is H, substituted acyl, linear or branched alkyl, wherein the alkyl can be interrupted by O, N, S heteroatoms; or R₂ and R₃ together represents O or S;
R₄ and R₅ each independently is H, substituted acyl, linear or branched alkyl, wherein the alkyl can be interrupted by O, N, or S heteroatoms; or R₄ and R₅ together represents O or S;
X₁, X₂, X₃, and X₄ can be identical or different, each of which independently is halogen atoms, hydroxyl, or linear or branched alkyl comprising 1 to 10 carbon atoms, or alkoxy, or acyloxy, and n is an integer of from 0 to 4;
C(1) and C(1') are of a stereoisomer configuration selected from RR, SS, 1S1'R, 1R1'S.

The bisbenzylisoquinoline alkaloid of Formula (I) can be selected from the following:

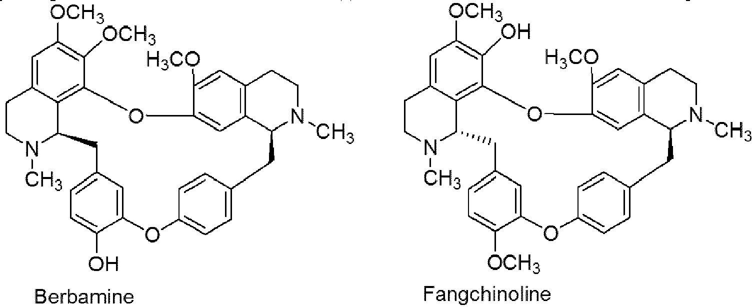

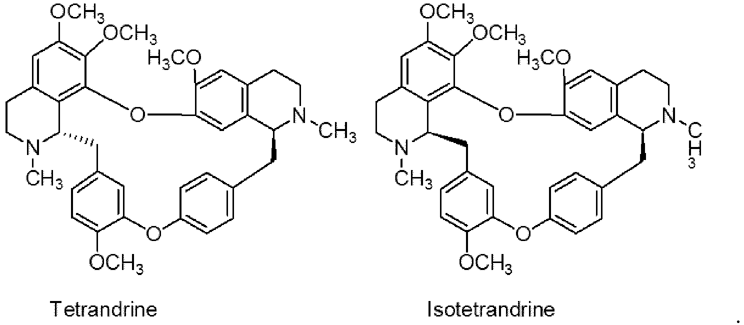

The bisbenzylisoquinoline alkaloid of Formula (I) can be prepared and characterized in structure and activity according to the methods described in, such as, CN1903856A, US 6617335 B1, or CN101371839A and characterized by the structure and activity thereof.

A suitable combination of the bisbenzylisoquinoline alkaloid of Formula (I) and the imatinib mesylate (II) of the present invention is used as an antitumor medicament. The synergistic effect between the two, particularly the pharmaceutical combination of berbamine and imatinib mesylate, as well as the pharmaceutical combination of tetrandrine and imatinib mesylate, achieves better therapeutic effects.

The bisbenzylisoquinoline alkaloid of Formula (I) can be present in the pharmaceutical combination in the form of its corresponding salt formed with a pharmaceutically acceptable inorganic or organic acid.

The term "aromatic hydrocarbyl" refers to an aryl without a heteroatom, including aryl, arylalkyl, and alkylaryl.

The term "aromatic heterocycyl" refers to an aromatic hydrocarbyl with a heteroatom, including heterocyclic aryl, heterocyclic arylalkyl, and alkylheterocyclic aryl. The heteroatom refers to N, O, and S. An arylheterocyclic radical can comprise one or more heteroatoms.

The term "halogen", "halo" or "hal" means fluorine, chlorine, bromine or iodine.

The term "C₁-C₆ substituted amino" refers to -N- C₁-C₆ alkyl and -N-C₃-C₆cycloalkyl.

The term "C₁-C₆ alkoxy" refers to -O- C₁-C₆ alkyl and -O-C₃-C₆cycloalkyl.

The term "C₁-C₆ alkylthio" refers to -S- C₁-C₆ alkyl and -S-C₃-C₆cycloalkyl.

The term "a pharmaceutically acceptable salt" includes, but not limited to, tosylate, methanesulfonate, malate, acetate, citrate, malonate, tartrate, succinate, benzoate, ascorbate, α-ketoglutarate, and α-glycerophosphate. Suitable inorganic salts may also be formed, including but not limited to, hydrochlorate, sulfate, nitrate, bicarbonate and carbonate, phosphate, hydrobromate, hydriodate salts and the like.

The terms "treatment," "treating," "treat," and the like used herein refer generally to obtaining a desired pharmacological and/or physiological effect. The effect may be prophylactic in terms of completely or partially preventing a disease or symptoms thereof and/or may be therapeutic in terms of partial or complete stabilization or cure of a disease and/or adverse effects caused by the disease. "Treatment" as used herein covers any treatment of a disease in a subject, including: (a) preventing the disease or symptoms from occurring in a subject who is predisposed to the disease or symptoms but has not yet been diagnosed as having it; (b) inhibiting the symptoms of a disease, i.e., arresting its development; or (c) relieving the symptoms of a disease, i.e., causing regression of the disease or symptoms.

The present invention also provides a suitable combination of the bisbenzylisoquinoline alkaloid of Formula (I) and the imatinib mesylate (II) as an antitumor medicament. The synergistic effect between the two, particularly the pharmaceutical combination of berbamine and imatinib mesylate, as well as the pharmaceutical combination of tetrandrine and imatinib mesylate, achieves better therapeutic effects.

The present invention relates to two or more combinations of the bisbenzylisoquinoline alkaloid of Formula (I) and the imatinib mesylate (II) as an antitumor medicament, whose composition and weight percentage can be as follows:
(1) berbamine comprises 0.1-80%, preferably 1-50%, of the active components; and
(2) imatinib mesylate comprises 0.1-80%, preferably 1-50%, of the active components.

The present invention relates to combinations of two or more of the bisbenzylisoquinoline alkaloid of Formula (I) and the imatinib mesylate (II) as an antitumor medicament, whose composition and weight percentage can also be as follows:
(1) tetrandrine comprises 0.1-80%, preferably 1-50%, of the active component; and
(2) imatinib mesylate comprises 0.1-80%, preferably 1-50%, of the active component.

The present invention provides pharmaceutical combinations that comprise at least two compounds as set forth above and optionally a pharmaceutically acceptable excipient.

The methods for preparing various pharmaceutical compositions having a certain amount of active components are known or will be apparent to those skilled in the art in light of this disclosure. As described in REMINGTON'S PHARMACEUTICAL SCIENCES, Martin, E.W., ed., Mack Publishing Company, 19th ed. (1995), the methods for preparing such pharmaceutical compositions include incorporation of other suitable pharmaceutical excipients, carriers, diluents, etc.

The pharmaceutical preparations of the present invention are produced by known methods, including routine mixing, dissolving, or lyophilizing processes.

The pharmaceutical combination of the present invention may be formulated into a pharmaceutical composition and administered to a patient in a route suitable for the selected administration manner, e.g., orally or parenterally (by an intravenous, intramuscular, topical or subcutaneous route).

Thus, the pharmaceutical combination of the present invention may be systemically administered, e.g., orally, in conjugation with a pharmaceutically acceptable carrier such as an inert diluent or an assimilable edible carrier. They may be enclosed in hard or soft gelatin capsules, or may be compressed into tablets. For oral therapeutic administration, the active compound may be combined with one or more excipients and may be taken in a form of ingestible tablet, buccal tablet, troche, capsule, elixir, suspension, syrup, wafer, and the like. Such a composition and preparation should contain at least 0.1% of the active compound. This proportion of the compositions and preparations may, of course, vary and may conveniently be from about 1% to about 99% by the weight of a given unit dosage form. The active compound is present in such a therapeutically useful composition in an amount such that an effective dosage level is achieved.

A tablet, troche, pill, capsule and the like may also comprises a binder such as gum tragacanth, acacia, corn starch or gelatin; an excipient such as dicalcium phosphate; a disintegrating agent such as corn starch, potato starch, alginic acid and the like; a lubricant such as magnesium stearate; and a sweetening agent such as sucrose, fructose, lactose or aspartame or a flavoring agent such as peppermint, wintergreen oil, or cherry flavor. When being a capsule as the unit dosage form, it may comprise, in addition to the above material types, a liquid vehicle such as a vegetable oil or polyethylene glycol. Various other materials may be present as coatings or otherwise modify the physical form of the solid unit dosage form. For instance, a tablet, pill, or capsule may be coated with gelatin, wax, shellac or sugar, etc. A syrup or elixir may contain an active compound, a sweetening agent such as sucrose or fructose, a preservative such as methylparaben or propylparaben, a dye and a flavoring agent (such as cherry or orange flavor). Of course, any materials used in preparing any unit dosage form should be pharmaceutically acceptable and substantially non-toxic in the amounts employed. In addition, the active compound may be incorporated into a sustained-release preparation and device.

The active compound may also be administered by infusion or injection intravenously or intraperitoneally. An aqueous solution of the active compound or its salt may be prepared, optionally mixed with a nontoxic surfactant. A dispersion can also be prepared in glycerol, liquid polyethylene glycol, triacetin, and a mixture thereof and in an oil. Under ordinary storage and use conditions, these preparations contain a preservative to prevent the growth of microorganisms.

The pharmaceutical dosage forms suitable for injection or infusion can include a sterile aqueous solution or dispersion or a sterile powder comprising the active ingredient (optionally encapsulated in liposomes) which are adapted for an extemporaneous preparation of a sterile injectable or infusible solution or dispersion. In all cases, the final dosage form must be sterile, liquid and stable under the manufacture and storage conditions. The liquid carrier or vehicle may be a solvent or a liquid dispersion medium comprising, for example, water, ethanol, a polyol (for example, glycerol, propylene glycol, liquid polyethylene glycol, and the like), a vegetable oil, a nontoxic glyceryl ester, and a suitable mixture thereof. The proper fluidity can be maintained, for example, by formation of liposomes, by maintenance of the required particle size in the case of dispersion or by the use of a surfactant. The prevention of microorganism action can be achieved by various antibacterial and antifungal agents, such as parabens, chlorobutanol, phenol, sorbic acid, thimerosal, and the like. In many cases, it will be preferable to include an isotonic agent, such as a sugar, a buffer agent or sodium chloride. Prolonged absorption of an injectable composition can be obtained by the use of a composition of the agents for delaying absorption, for example, aluminum monostearate and gelatin.

A sterile injectable solution is prepared by combining the active compound in a required amount in a suitable solvent with various additional components as listed above as required, followed by filter sterilization. In the case of sterile powder for preparation of a sterile injectable solution, the preferred preparation process is the vacuum drying and lyophilization techniques, which yield a powder of the active ingredient plus any additional desired ingredient present in the previously sterile-filtered solution.

Useful solid carriers include finely divided solids such as talc, clay, microcrystalline cellulose, silica, alumina and the like. Useful liquid carriers include water, ethanol or ethylene glycol or a water-ethanol/ ethylene glycol mixture, in which the compound of the present invention can be dissolved or dispersed at an effective level optionally with the aid of a non-toxic surfactant. An adjuvant (such as a flavour) and an additional antimicrobial agent can be added to optimize the properties for a given application.

A thickening agent (such as a synthetic polymer, a fatty acid, a fatty acid salt and ester, a fatty alcohol, a modified cellulose or a modified mineral) can also be used with a liquid carrier to form a spreadable paste, gel, ointment, soap and the like for application directly to the skin of a user.

The treatment required amount of the compound or an active salt or derivative thereof will vary depending not only on the selected particular salt but also on the administration route, the nature of the condition to be treated and the age and condition of the patient, and will be ultimately determined at the discretion of the attendant physician or clinician.

The above formulations can be present in a unit dosage form which is a physically discrete unit containing a unit dosage suitably administrating to a human or other mammalians. The unit dosage form may be a capsule or a tablet, or a plurality of capsules or tablets. Depending upon the intended particular treatment, the amount of the active ingredient in a unit dosage form can be varied or adjusted in the range of about 0.1 mg to about 1,000 mg or more.

If the pharmaceutical composition of the present invention is made into a formulation and administered intestinally or non-intestinally, then each formulation can have the active components of the following contents, calculated based on basic unit of measurement:
(a) bisbenzylisoquinoline alkaloid: 0.1 - 150 mg;
(b) imatinib mesylate: 0.1 - 150 mg.
For example,
(a) berbamine: 0.1 - 150 mg;
(b) imatinib mesylate: 0.1 - 150 mg;
or alternatively,
(a) tetrandrine: 0.1 - 150 mg;
(b) imatinib mesylate: 0.1 - 150 mg.

In the formulation of the pharmaceutical composition of the present invention, the content of each main component can be, but not limited to, the following:
bisbenzylisoquinoline alkaloid: 1-50%;
imatinib mesylate: 1-50%;
starch: 1-45%;
dextrin: 0-20%;
microcrystalline cellulose: 0-25%;
sodium carboxymethyl starch: 0-5%;
polyethylene glycol: 0-5%;
hydroxypropyl methyl cellulose: 0-5%;
magnesium stearate: 0-5%.

The present invention also provides the use, particularly the antitumor use, of the pharmaceutical composition of the present invention. Correspondingly, the present invention provides a method for treating a subject suffering from tumor, comprising administrating to the patient in need thereof an effective amount of the pharmaceutical combination of the present invention. The pharmaceutical combination of the bisbenzylisoquinoline alkaloid of Formula (I) and the imatinib mesylate (II) of the present invention can be used to treat, for example, leukemia, multiple myeloma, lymphoma, liver cancer, gastric cancer, breast cancer, cholangiocellular carcinoma, pancreatic cancer, lung cancer, colorectal carcinoma, osteosarcoma, melanoma, human cervical cancer, glioma, nasopharyngeal carcinoma, laryngeal carcinoma, esophageal cancer, middle ear tumor, prostate cancer, among other tumors.

In the following examples, the present invention will be explained in more detail. However, it should be understood that the following examples are intended to illustrate the present invention but not to limit the scope of the present invention in any way.

The raw chemicals used in the following examples are commercially available.

### Example

### Example 1: Pharmaceutical combination of bisbenzylisoquinoline alkaloid and imatinib mesylate for the inhibition of leukemia cells in vitro

### Experiment 1: Pharmaceutical combination of berbamine (BBM) / tetrandrine (TTD) and imatinib mesylate (IM) for the inhibition of leukemia cells in vitro

### (1) Experimental materials

Leukemia cell lines: K562 (chronic myeloid leukemia cell line), K562/ADR (doxorubicin(ADM)-resistant human chronic granulocytic leukemia cell line), KCL-22 (chronic myeloid leukemia cell line), KCL-22 M (human chronic granulocytic leukemia cell line mutant), CML-BC (KCL-28 patient's cells), all of which are donated by Cancer Research Institute of Zhejiang University, China; KU812 (chronic myeloid leukemia cell line), purchased from Fuxiang Bio-tech Co. Ltd., Shanghai, China.
(2) Reagents: berbamine (BBM, purchased from Pukang Biochem Co., Ltd., Shifang, Sichuan); tetrandrine (TTD, purchased from Nanjing Zelang Pharmaceutical Science & Technology Co., Ltd.); imatinib mesylate (IM, purchased from Wuyi Jiayuan Pharmaceutical Materials Co., Ltd.)
(3) Main apparatuses:
   Cell incubator (Thermo Scientific 3111)
   Biosafety cabinet (Heal Force, Hfsafe-1200A2)
   Microplate reader (Bio-rad, Imark)

### (4) Experimental method

1. Determine IC₅₀ value (µg/mL) after 72 hours of separate compounds on leukemia cells in vitro. Well-growing leukemia cells were obtained and inoculated into wells of a 96-well cell culture plate at 5000 cells/well. Respective compounds of various concentrations were added, mixed uniformly, and incubated (5% CO₂) at 37°C for 72 hours. Then the cell viability was determined by the MTT method. In this experiment, the cell viability in control group (not treated with any compound) was set as 100%, and the half maximal inhibitory concentrations of BBM or TTD and IM for the leukemia cell growth at 72 hours (IC₅₀ value of 72 hours, µg/mL) were calculated.
2. Determine the inhibitory effects of bisbenzylisoquinoline in combination with IM on leukemia cells in vitro. The pretreatment of the cells was the same as in a). The control group was not treated with the compound while administered. In the experimental group, compounds were added respectively to the concentration under which the IC₅₀ (µg/mL) of the compounds in step 1 achieves. As to the combination group, the two compounds were added together into the same group of wells. After 72 hours of incubation, the MTT method was used for the determination of cell viability. The cell viability of the control group wa set to 100% in this experiment, and the BBM/TTD, IM single drug and combined effects on cell viability (100%) were calculated.

### (5) The experimental results

### 1. The effect of pharmaceutical combination of BBM and IM on leukemia cells

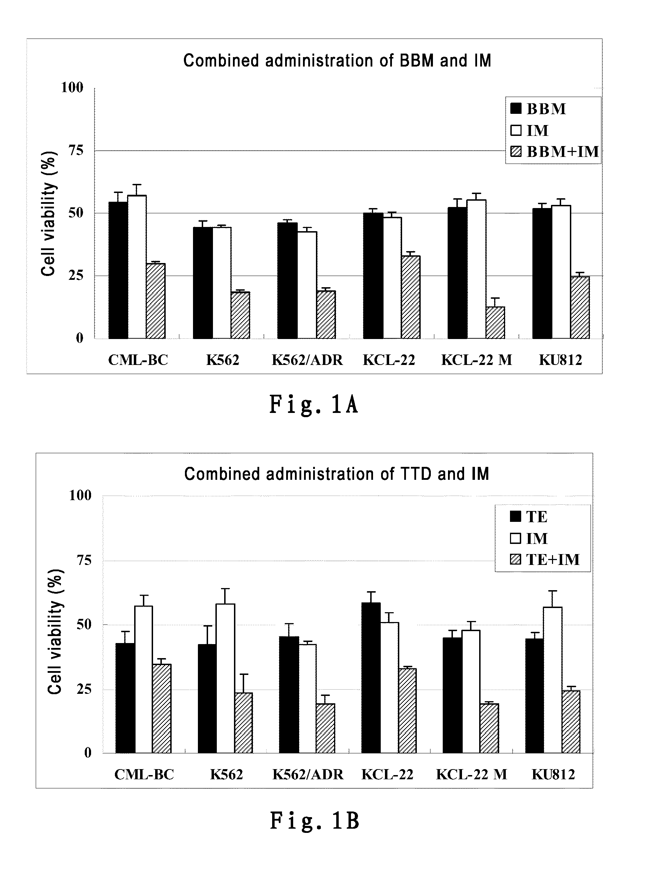

As is seen from Table 1 and Figure 1A, for the leukemia cell lines tested, the IC₅₀ dose of the pharmaceutical combination results in cell viability that is more than 10% lower than either BBM or IM used alone. In addition, in the case of KCL-22 M, after the combined administration of BBM and IM at IC₅₀ dosage, the cell viability is reduced by more than 40% as compared with single drug.

### 2. The effect of pharmaceutical combination of TTD and IM on leukemia cells

As is seen from Table 2 and Figure 1B, for the leukemia cell lines tested, the IC₅₀ dose of the pharmaceutical combination results in significantly lower cell viability than either TTD or IM used alone. In addition, in the case of KCL-22 M, after the combined administration of TTD and IM at IC₅₀ dosage, the cell viability is reduced by almost 30% as compared with single drug.

**Table 1: The effect of pharmaceutical combination of BBM and IM on leukemia cells**

| Cell lines | BBM | | | IM | | | BBM+IM | | |
|---|---|---|---|---|---|---|---|---|---|
| | Dosage (µg/mL) | CV(%) | SD | Dosage (µg/mL) | CV(%) | SD | Dosage (µg/mL) | CV(%) | SD |
| CML-BC | 1.5 | 54.44 | 3.75 | 0.02 | 57.07 | 4.28 | 1.5+0.02 | 29.7 | 0.81 |
| K562 | 1.6 | 44.47 | 2.48 | 0.03 | 44.25 | 1.12 | 1.6+0.03 | 18.45 | 0.68 |
| K562/ADR | 1.5 | 46.27 | 1.24 | 0.2 | 42.34 | 2.1 | 1.5+0.2 | 18.92 | 1.05 |
| KCL-22 | 6.5 | 50 | 1.77 | 0.08 | 48.45 | 2.11 | 6.5+0.08 | 32.7 | 1.9 |
| KCL-22 M | 1.7 | 52.39 | 3.49 | 4.6 | 55.17 | 2.7 | 1.7+4.6 | 12.55 | 3.56 |
| KU812 | 1.5 | 51.59 | 2.26 | 0.03 | 53.26 | 2.58 | 1.5+0.03 | 24.59 | 1.59 |

**Table 2: The effect of pharmaceutical combination of TTD and IM on leukemia cells**

| Cell lines | TE | | | IM | | | TE+IM | | |
|---|---|---|---|---|---|---|---|---|---|
| | Dosage (µg/mL) | CV(%) | SD | Dosage (µg/mL) | CV(%) | SD | Dosage (µg/mL) | CV(%) | SD |
| CML-BC | 0.3 | 42.59 | 4.94 | 0.01 | 57.13 | 4.4 | 0.3+0.01 | 34.5 | 2.44 |
| K562 | 0.86 | 42.48 | 7.09 | 0.02 | 57.91 | 6.33 | 0.86+0.02 | 23.56 | 7.01 |
| K562/ADR | 0.4 | 45.28 | 5.31 | 0.16 | 42.52 | 1.02 | 0.4+0.16 | 19.14 | 3.56 |
| KCL-22 | 1.48 | 58.74 | 4.04 | 0.05 | 51.06 | 3.55 | 1.48+0.05 | 33.11 | 0.78 |
| KCL-22 M | 1.2 | 44.96 | 2.76 | 3.27 | 48.02 | 3.24 | 1.2+3.27 | 19.07 | 1.11 |
| KU812 | 0.80 | 44.54 | 2.63 | 0.01 | 56.7 | 6.43 | 0.8+0.01 | 24.24 | 1.72 |

| | | | | | | | | | |
|---|---|---|---|---|---|---|---|---|---|
| Note: CV means cell viability. | | | | | | | | | |

### Example 2: Pharmaceutical combination of bisbenzylisoquinoline alkaloid and imatinib mesylate for the inhibition of leukemia transplanted tumor

### Experiment 2-1: Pharmaceutical combination of berbamine (BBM) and imatinib mesylate (IM) for the inhibition of leukemia transplanted tumor in BALB/c-nu nude mice

### (1) Experimental materials

Leukemia cell lines: K562/ADR (ADM-resistant human chronic granulocytic leukemia cell line); Animals: BALB/c-nu nude mice
(2) Reagents: berbamine (BBM, purchased from Pukang Biochem Co., Ltd., Shifang, Sichuan); imatinib mesylate (IM, purchased from Wuyi Jiayuan Pharmaceutical Materials Co., Ltd.)
(3) Main apparatuses:
   Cell incubator (Thermo Scientific 3111)
   Laminar flow rack (Suhang Brand clean animal breeding cabinet, Type DJ-2).

### (4) Experimental method

Under sterile conditions, the above tumor cells were collected in the logarithmic growth phase, and were injected by subcutaneous injection in an amount of 1 × 10⁷/0.2 mL/nude mice (cell viability>95%) into the right subaxillary of the nude mice, thus establishing leukemia transplated tumor models of in nude mice. The mice were administered from the third day after the inoculation. The treated group was intragastrically administered at the experimentally designed amount, the negative control group was intragastrically administered with sterile water, and the positive control group was intragastrically administered with IM. Each mouse was intragastrically administered in 0.4 ml each time and 2 times a day. The administrations were successive for 14 days. The day before administration was deemed as Day 0 and the body weight and tumor size of the mice were determined every 5 days to produce a dynamic plot on body weight and tumor growth. On Day 23, the mice were sacrificed and the tumors were taken out and weighed. The tumor inhibition rate (%) was calculated after the effect of the medicament based on a tumor inhibition rate of the control group being zero.

### (5) The experimental results

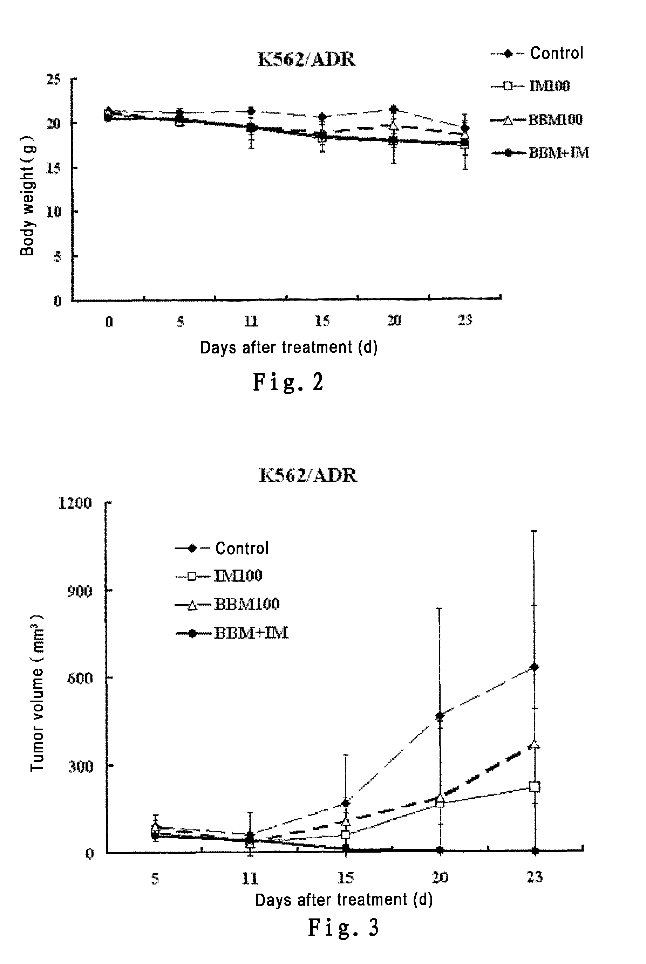

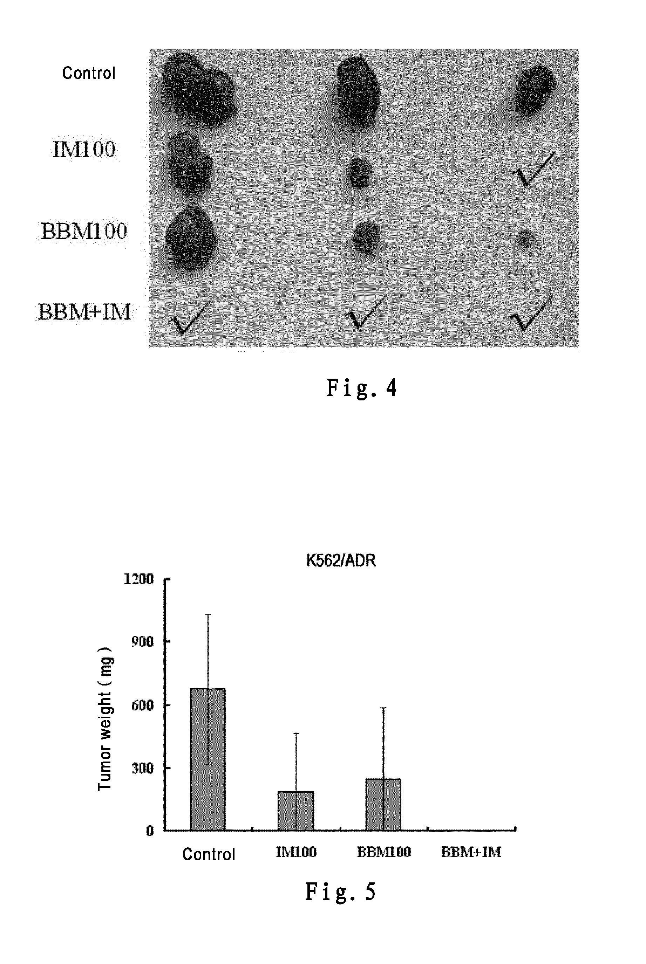

This experiment tests the effect of BBM and IM on leukemia transplated tumor. As is seen from Table 3 and Figures 3 to 6, the combination of BBM and IM improves the tumor inhibition rate by more than 28% as compared with single drugs, and achieves a tumor inhibition rate up to 100% as compared with the control groups. According to the initial and final body weights, the combination of the two drugs did not cause more reduction in body weight than using IM alone, thus indicating that such combination does not further increase toxicity (see Figure 2).

**Table 3: The effect of the pharmaceutical combination of BBM and IM on the leukemia transplanted tumor in BALB/c-nu nude mice (x±s)**

| Groups | Dosage (mg/kg) | No. of Animals | | Body weight (g) | | Tumor weight (g) | Tumor inhibition rate (%) |
|---|---|---|---|---|---|---|---|
| | | Initial | Final | Initial | Final | | |
| Control | -- | 3 | 3 | 21.43±0.15 | 19.30±0.56 | 0.68±0.36 | -- |
| IM | 100 | 3 | 3 | 21.00±0.12 | 17.37±2.75 | 0.19±0.27 | 71.98 |
| BBM | 100 | 3 | 3 | 21.23±0.23 | 18.57±2.28 | 0.25±0.34 | 63.66 |
| BBM+IM | 100+100 | 3 | 3 | 20.63±0.06 | 17.63±1.46 | 0* | 100 |

| | | | | | | | |
|---|---|---|---|---|---|---|---|
| Note: * denotes P<0.05 as compared with the sterile water control groups | | | | | | | |

### Experiment 2-2: Pharmaceutical combination of tetrandrine (TTD) and imatinib mesylate (IM) for the inhibition of leukemia transplanted tumor in BALB/c-nu nude mice

(1) Experimental materials
   Leukemia cell lines: K562/ADR (ADM-resistant human chronic myeloid leukemia cell line); Animals: BALB/c-nu nude mice
(2) Reagents: tetrandrine (TTD, purchased from Nanjing Zelang Pharmaceutical Science & Technology Co., Ltd.);
   imatinib mesylate (IM, purchased from Wuyi Jiayuan Pharmaceutical Materials Co., Ltd.).
(3) Main apparatuses: Cell incubator; laminar flow rack.
(4) Experimental method

Under sterile conditions, the above tumor cells were collected in the logarithmic growth phase, and were injected by subcutaneous injection in an amount of 1 × 10⁷/0.2 mL/nude mice (cell viability>95%) into the right subaxillary of the nude mice, thus establishing leukemia transplated tumor models of in nude mice. The mice were administered from the sixth day after the inoculation. The treated group was intragastrically administered at the experimentally designed amount, the negative control group was intragastrically administered with sterile water, and the positive control group was intragastrically administered with IM. Each mouse was intragastrically administered in 0.4 ml each time and 2 times a day. The administrations were successive for 14 days. The day before administration was deemed as Day 0 and the body weight and tumor size of the mice were determined every 5 days to produce a dynamic plot on body weight and tumor growth. On Day 20, the mice were sacrificed and the tumors are taken out and weighed. The tumor inhibition rate (%) was calculated after the effect of the medicament based on a tumor inhibition rate of the control group being zero.

### (5) The experimental results

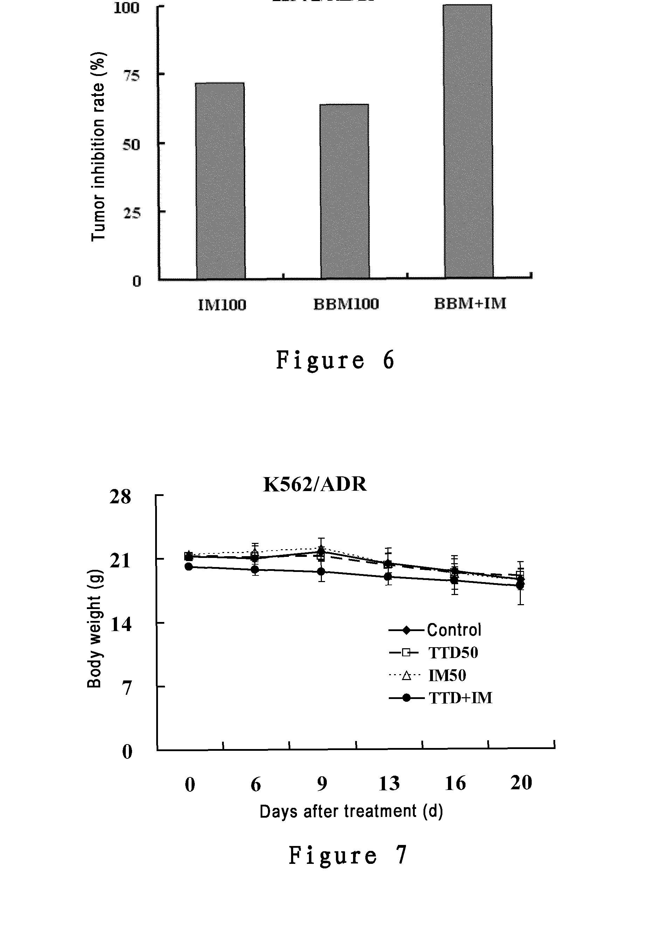

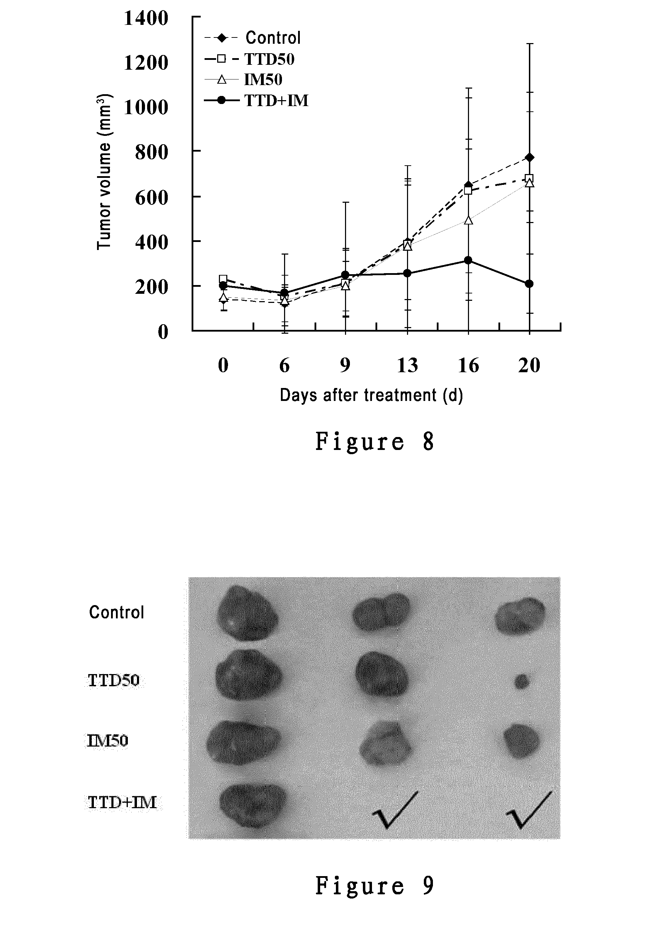

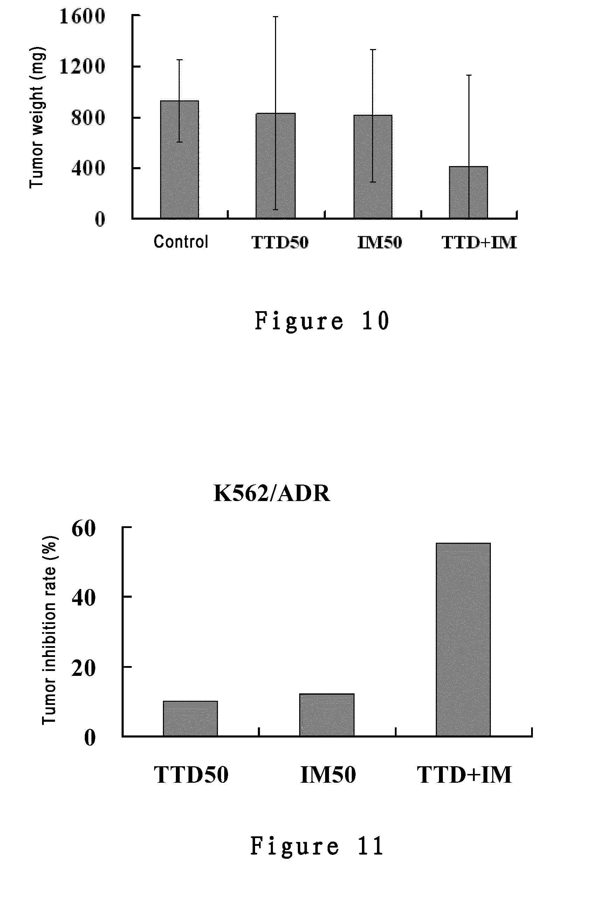

This experiment tested the effect of TTD and IM on leukemia transplated tumor. As is seen from Table 4 and Figures 8 to 11, the combination of TTD and IM improves the tumor inhibition rate by more than 43% as compared with single drugs, and achieves a tumor inhibition rate up to 55% as compared with the control groups. According to the initial and final body weights, the combination of the two drugs did not lead to more reduction in body weight than the use of IM alone, thus indicating that such combination does not further increase toxicity (see Figure 7).

**Table 4: The effect of the pharmaceutical combination of TTD and IM on the leukemia transplanted tumor weight in BALB/c-nu nude mice (x±s)**

| Groups | Dosage (mg/kg) | No. of Animals | | Body weight (g) | | Tumor weight (g) | Tumor inhibition rate (%) |
|---|---|---|---|---|---|---|---|
| | | Initial | Final | Initial | Final | | |
| Control | -- | 3 | 3 | 21.23±0.40 | 18.50±1.13 | 0.93±0.32 | -- |
| TTD | 50 | 3 | 3 | 21.30±0.10 | 18.93±1.53 | 0.83±0.76 | 10.18 |
| IM | 50 | 3 | 3 | 21.47±0.21 | 18.40±0.52 | 0.81±0.52 | 12.26 |
| TTD+IM | 50+50 | 3 | 3 | 20.10±0.10 | 17.80±2.00 | 0.41±0.72 | 55.27 |

## Claims

1. An antitumor pharmaceutical combination, comprising a bisbenzylisoquinoline alkaloid of Formula (I) or a pharmaceutically acceptable salt thereof and imatinib (II) or a pharmaceutically acceptable salt thereof as an active comnonent 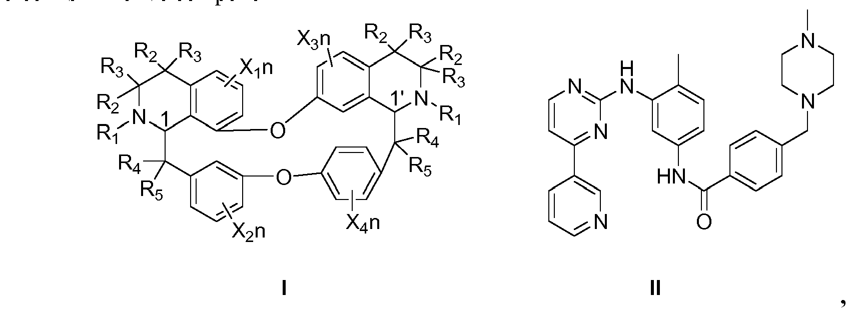 in Formula (I),
R₁ is H or linear or branched alkyl comprising 1 to 10 carbon atoms;
R₂ and R₃ each independently is H, substituted acyl, linear or branched alkyl, wherein the alkyl can be interrupted by O, N, or S heteroatoms; or R₂ and R₃ together represents O or S;
R₄ and R₅ each independently is H, substituted acyl, linear or branched alkyl, wherein the alkyl can be interrupted by O, N, or S heteroatoms; or R₄ and R₅ together represents O or S;
X₁, X₂, X₃, and X₄ can be identical or different, each of which independently is halogen atom, hydroxyl, or linear or branched alkyl comprising 1 to 10 carbon atoms, or alkoxy, or acyloxy, and n is an integer of from 0 to 4;
C(1) and C(1') are of a stereoisomer configuration selected from RR, SS, 1S1'R, 1R1'S.

2. The antitumor pharmaceutical combination according to claim 1, wherein the pharmaceutically acceptable salt of imatinib is mesylate thereof.

3. The antitumor pharmaceutical combination according to claim 1 or 2, wherein the bisbenzylisoquinoline alkaloid of Formula (I) is berbamine.

4. The antitumor pharmaceutical combination according to claim 1 or 2, wherein the bisbenzylisoquinoline alkaloid of Formula (I) is tetrandrine.

5. The antitumor pharmaceutical combination according to any one of claims 1 to 4, **characterized in that** the bisbenzylisoquinoline alkaloid of Formula (I) or a pharmaceutically acceptable salt thereof comprises 0.1-99.9% of the total weight of the active components; and imatinib or a pharmaceutically acceptable salt thereof comprises 0.1-99.9% of the total weight of the active component.

6. The antitumor pharmaceutical combination according to any one of claims 1 to 4, **characterized in that** the bisbenzylisoquinoline alkaloid of Formula (I) or a pharmaceutically acceptable salt thereof comprises 1-99% of the total weight of the active components; and imatinib or a pharmaceutically acceptable salt thereof comprises 1-99% of the total weight of the active components.

7. The antitumor pharmaceutical combination according to any one of claims 1 to 4, **characterized in that** the bisbenzylisoquinoline alkaloid of Formula (I) or a pharmaceutically acceptable salt thereof comprises 10-90% of the total weight of the active components; and imatinib or a pharmaceutically acceptable salt thereof comprises 10-90% of the total weight of the active components.

8. The antitumor pharmaceutical combination according to any one of claims 1 to 4, **characterized in that** the bisbenzylisoquinoline alkaloid of Formula (I) or a pharmaceutically acceptable salt thereof comprises 20-80% of the total weight of the active components; and imatinib or a pharmaceutically acceptable salt thereof comprises 20-80% of the total weight of the active components.

9. Use of the bisbenzylisoquinoline alkaloid of Formula (I) or a pharmaceutically acceptable salt thereof and imatinib or a pharmaceutically acceptable salt thereof as recited in any of claims 1-8 as the active component in the manufacture of an antitumor medicament.

10. A method for treating a subject suffering from tumor, comprising administrating to the subject in need thereof an effective amount of the bisbenzylisoquinoline alkaloid of Formula (I) or a pharmaceutically acceptable salt thereof and imatinib or a pharmaceutically acceptable salt thereof as recited in any of claims 1-8.

11. The pharmaceutical combination according to any of claims 1-8 for use as an antitumor agent.

12. The use, method or pharmaceutical combination according to claim 9, 10 or 11, wherein the tumor is selected from leukemia, multiple myeloma, lymphoma, liver cancer, gastric cancer, breast cancer, cholangiocellular carcinoma, pancreatic cancer, lung cancer, colorectal carcinoma, osteosarcoma, human cervical cancer, glioma, nasopharyngeal carcinoma, laryngeal carcinoma, esophageal cancer, middle ear tumor, melanoma, ovarian carcinoma, renal carcinoma, metrocarcinoma, osteosarcoma, and prostate cancer.
